# EUROPEAN PATENT SPECIFICATION

(11) **EP 2 819 585 B1**
(45) Date of publication and mention of the grant of the patent: **29.11.2017**
(21) Application number: 13707610.5
(22) Date of filing: 28.02.2013
(51) Int. Cl.: A61B 17/00

(54) **A DEVICE FOR OCCLUDING AN OPENING IN A BODY AND ASSOCIATED METHODS**
VORRICHTUNG ZUM VERSCHLIESSEN EINER ÖFFNUNG IN EINEM KÖRPER UND ZUGEHÖRIGE VERFAHREN
DISPOSITIF D'OCCLUSION D'UNE OUVERTURE DANS UN CORPS ET MÉTHODES ASSOCIÉES

(30) Priority: 29.02.2012 US 201261605102 P; 27.09.2012 US 201261706698 P
(43) Date of publication of application: 07.01.2015
(73) Proprietor: Occlutech Holding AG, 8201 Schaffhausen (CH)
(72) Inventor: AKPINAR, Mehmet Hakan, Kadikoy, Istanbul 34710 (TR)
(74) Representative: KIPA AB
(86) International application number: PCT/EP2013/054112
(87) International publication number: WO 2013/152891

(56) References cited:
- WO-A2-99/38454
- DE-A1-102010 019 365
- US-A- 5 976 174
- US-A1- 2002 183 787
- US-A1- 2005 222 488
- US-A1- 2006 009 800
- US-A1- 2008 051 830
- US-A1- 2010 211 046

## Description

### Field of the Invention

This disclosure pertains in general to the field of medical implants or endoprostheses. More particularly the disclosure relates to a device for occluding an opening in a body and associated methods.

### Background of the Invention

The present disclosure is related to the sealing of an opening in a body vessel or the wall of a heart cavity, e.g. a blood vessel or a human heart, and more precisely to a device for occluding an opening in a body, and a method for manufacturing such a device.

There are holes and openings not created by interventional treatment activity, acquired as a result of disease or congenital. Some products for closing acquired or congenital defects are devices having umbrella shaped discs with spikes and a covering cloth. One disc is placed on one side of the opening to be closed and another disc is placed on the other side of the opening to be closed. Thereafter, the discs are pressed against each other and locked to seal the opening. StarFlex® (NMT Medical Inc®, Boston MA) and CARDIA Patent Foramen Ovale Closure Device® (Cardia Inc®, Burnsville, Minnesota) are such devices.

Other devices are made of Nitinol threads and have a double disc shape with a waist between the discs. They are inserted in openings that are to be closed, one disc on each side of the hole that are to be closed and the waist in the center of the hole, the discs being larger than the hole. There are two examples of such devices. The first, made by Occlutech® having one fixation point at the end of the device and the second, made by AGA medical® having two fixation points, one at each end of the device. In these devices, the Nitinol threads are joined in the centre of one or both of the discs. These devices work well when the hole or opening is circular and the wall of the opening is thin..

However, some defects, e.g. ventricular septal defects, are not circular holes or openings. Instead there may be ruptures, for instance with torn tissue. Ventricular walls are substantially thicker tissue structures than septal walls. Such ruptures in structures like these may for instance occur, after a myocardial infarct or as part of a muscular ventricular septal defect.

When using a prior art occluder for closing one of the passages of such complicated defects, it may be difficult to completely seal such a gap of an opening, thus the prior art devices do not always work well with these kind of ruptures, since they sometimes may further tear tissue that has already been torn, and thus may increase the rupture size.

Moreover, when it comes to ruptures, the prior art devices may not always cover all of the shunts or channels.

Furthermore, the prior art devices may for some defects, such as ruptures, cover an unnecessary large portion of healthy tissue. This may sometimes be undesired from a necrosis point of view.

WO2013152891 discloses a device including a frame defining a lumen, where the frame expands from a first configuration to a second configuration larger than the first configuration.

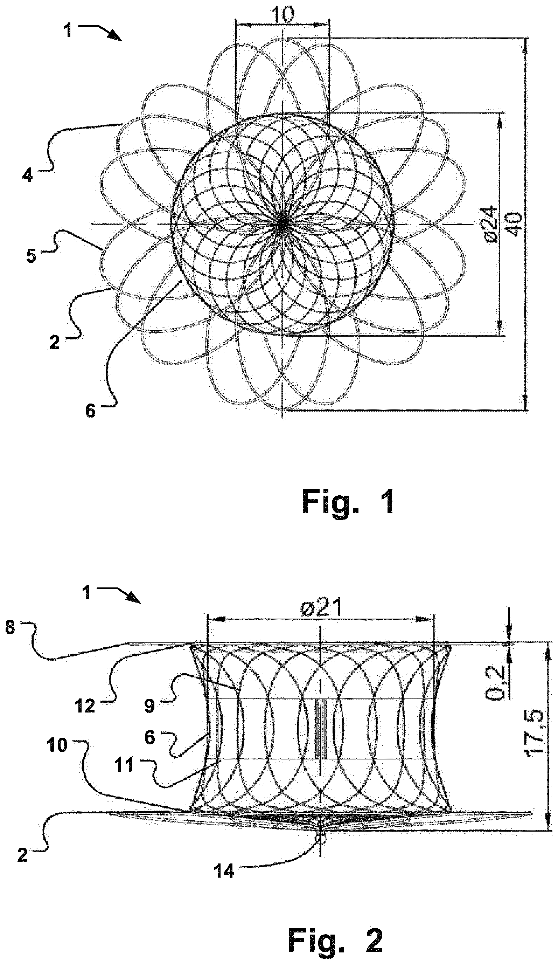

FIG. 1 illustrates the frame having a uniform cylindrical shape with a circular cross section. The frame can have cross sections that are elliptical, flattened circular, rectangular, or the like.

DE102010019365 discloses a bioabsorbable occlusion device, which is introduced by a catheter in a folded condition in a patient's body, where the occlusion device in an area of its surrounding envelope comes to a constriction, which on one side causes the anchoring, as the proximal and distal retention areas are not affected by this constriction and alternatively, the defect is closed so that at the conclusion of the implantation process a variable final shape is formed, which no longer coincides with the permanent initial form of the occlusion device.

Thus, there is a need of an improved device and/or associated method or procedure, which works well with ruptures and does not increase the rupture size.

There is also a need of an improved device and/or associated method or procedure, which can completely fill the gap of a rupture to eliminate residual shunts.

Furthermore, there may also be a need of an improved device and/or associated method or procedure, which covers healthy tissue to a less extent than prior art devices.

### Summary of the Invention

The invention relates to a device for occluding an opening in the heart according to claim 1 and to a method of manufacturing said device according to claim 8.The further aspects of the invention are defined in the dependent claims. None of procedures for occluding an opening in the body or heart which are disclosed in the following description, form part of the present invention. A disadvantage with the prior known devices for occluding an opening in a body is that when the device has a single body, the middle section, which can be called the waist, is circular and this middle section gets a lot of support from the right and left disc-shaped end sections in order to keep its shape circular, while trying to keep its shape memory. The reason that the middle section gets a lot of support from the end sections is that the different sections are all part of one integrated unit, produced the same way and comprising the same material, with the same size. Thereby the design is not flexible enough. The present disclosure overcomes this and/or other disadvantages with prior art by providing a device for occluding an opening in a body, in which device a second element of the device can be made more flexible than a first element and thus provide for an elastic design suitable for both systolic and diastolic thickness of the ventricular septum.

Some examples of the disclosure provide for a decreased material cost.

Some examples of the disclosure provide for less coverage of healthy tissue.

Some examples of the disclosure enable that the first and second elements can be manufactured in different ways independent of each other and thus with completely different properties, materials or size, such as diameter of thread or wire.

Some examples of the disclosure also provide for a second element, made of braided material, which can be made more flexible and thereby the device is made less prone to increase the rupture size of an opening, and the device can fill the gap completely to eliminate residual shunts or channels.

Some examples of the disclosure also provide for there being need for only very little radial strength in a second element of the device.

Some examples of the disclosure provide for a second element of the device, which has a lower expansive force than a first element.

Some examples of the disclosure provide for a second element of the device, which second element, and device, does neither tear a body opening nor further tear an already torn body opening.

Some examples of the disclosure provide for the assembling of a device from different, separately designed elements, so that the strength of the disc-shaped section of the first element does not affect the strength of the second element and the second element can stay oval with a very small retention force, caused by the shape memory.

Some examples of the disclosure provide for an elastic design, which works well with both sistolic and diastolic thickness of the ventricular septum.

Some examples of the disclosure enable both of the disc-shaped sections of a first element and a third element to be larger, i.e. have a larger diameter, and still be made up of less material, which is advantageous, since the material cost will be decreased and since healthy tissue will not be covered by the device to the same extent as prior art devices.

Some examples of the disclosure provide for allowing a sliding movement of the first element relative the second element.

Some examples of the disclosure also provide for enhancing the adhesion and proliferation of fibroblasts and endothelial cells onto surfaces.

Some examples of the disclosure also provide for overcoming certain materials cytotoxic issues.

Some examples of the disclosure also provide for faster endothelization.

Some examples of the disclosure also provide for a symmetrical design

Some examples of the disclosure also provide for a simplified manufacturing.

It should be emphasized that the term "comprises/comprising" when used in this specification is taken to specify the presence of stated features, integers, steps or components but does not preclude the presence or addition of one or more other features, integers, steps, components or groups thereof.

A ventricular septal defect is a defect in the ventricular septum, the wall dividing the left and right ventricles of the heart. The ventricular septum consists of an inferior muscular and superior membranous portion and is extensively innervated with conducting cardiomyocytes.

A muscular ventricular septal defect is a defect of the inferior muscular portion.

A postinfarction ventricular septal defect or myocardial infarction complications may occur immediately following a heart attack, e.g. a rupture of the intraventricular septum, the muscle separating the left and right ventricles, causing a ventricular septal defect with shunting of blood through the defect from the left side of the heart to the right side of the heart, which can lead to right ventricular failure as well as pulmonary overcirculation.

Perimembranous ventricular septal defects are located in the left ventricle outflow tract beneath the aortic valve.

Membranous ventricular septal defects are more common than muscular ventricular septal defects, and are the most common congenital cardiac anomaly.

Specific devices and methods described herein are applicable to treating at least one of the aforementioned defects, amongst others.

### Brief Description of the Drawings

Figure 1 is a top view in which an example of a device is illustrated for occluding an opening in a body.
Figure 2 is a lateral view in which an example of the device is illustrated for occluding an opening in a body.
Figure 3 is an elevated view illustrating an example of the device for occluding an opening in a body from the top at an angle.
Figure 4 is another view in which the device is illustrated for occluding an opening in a body, seen from the top at an angle.
Figure 5 is an elevated view in which an example of a part of a first element of a device for occluding an opening in a body from the top at an angle is illustrated.
Figure 6 is an elevated view in which an example of a first element of a device for occluding an opening in a body is illustrated from the top at an angle.
Figure 7 is another elevated view in which an example of a device for occluding an opening in a body is illustrated from the top at an angle.
Figure 8 is yet another view in which an example of a device for occluding an opening in a body is illustrated from the top at an angle.
Figure 9 is a flowchart in which steps of an exemplary method of manufacturing a device for occluding an opening in a body is illustrated.
Figure 10 is a view in which an example of a device is illustrated, for occluding an opening in a body, in its at least partly collapsed state.
Figure 11 is a schematic view in which examples of catheters for a medical procedure are illustrated.
Figure 12 is a lateral view in which an example of a device for occluding an opening in a body is illustrated.
Figure 13 is a top view in which a second element of an example of a device for occluding an opening in a body is illustrated.
Figure 14 is another top view in which an example of a second element of a device for occluding an opening in a body is illustrated.
Figure 15 is a lateral view in which an example of a device for occluding an opening in a body is illustrated.

### Description of examples

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on an example of the present disclosure applicable to occlusion of a body opening and in particular to a device for occluding a body opening, such as a body opening associated with a postinfarctation ventricular septal defect. However, it will be appreciated that the disclosure is not limited to this application but may be applied to many other situations, including for example occlusion of a body opening associated with a perimembranous ventricular septal defect or a muscular ventricular septal defect.

In an example of the disclosure according to Figure 1, a device 1 is provided with a first element 2. The element 2 comprises at least one thread, strand or wire 3, i.e. the element can be made up of one sufficiently long thread or wire, but it can also be made up of a plurality of threads or wires, that have been put together to form the element 2. The element 2 also comprises a first section 4, which section 4 is normally not braided, but shaped like a disc, i.e. extending substantially in one geometrical plane. The first section 4 comprises at least one loop 5 of the thread or wire 3 extending from a central portion of the first section 4 to a peripheral portion of the first section 4 and returning back to the central portion.

By forming the first element this way, instead of forming it from braided material as it has been done in prior art, the first element can be larger and still be made up of less material than prior art, which is advantageous, since the material cost will be decreased and since healthy tissue will not be covered by the device to the same extent as prior art devices.

The exemplary device 1 illustrated in Figs. 1 and 2 with sizing measures. The dimension of the sizing measures is [mm]. Relative dimensions of the example can thus be determined from the drawings if so desired. However, the absolute size measures are only to be seen as exemplary numbers of a specific example of a device 1.

The device 1 is also provided with a second element 6, such as a shaft section. The second element 6 shown in Figure 1 is also shown from the side in Figure 2. The second element 6 comprises a braided structure. The braided structure comprises and is made of at least one braided thread or wire 9, i.e. it can be made up of one sufficiently long thread or wire, but it can also be made up of a plurality of threads or wires. The second element 6 is at least in a longitudinal section 11 tubular and may be substantially circular or alternatively oval and comprises three different sections, one first section 10 at the end of the element, one second section 11, which is tubular and circular or may be oval, in the middle and one third section 12 at the other end of the second element 6. In some examples, a cross-sectional dimension, such as the diameter, of the second element 6 and/or the second section 11 thereof is of the same size, substantially the same size or smaller than the opening to be occluded by the device 1 for all longitudinal sections, i.e. first, second and third sections 10, 11, 12 of the second element 6, while the device is in a preset, expanded configuration. The longitudinal section 11 of the second element 6 can have a non-concentric oval or elliptic shape in a cross-section perpendicular to a centre axis of the longitudinal section. Due to this feature, a more flexible movement in a longitudinal direction is provided. Thus, the device 1 more easily moves with the movement of the surrounding tissue, i.e. with the movement according to the systole and the diastole. Thus, the device 1 adapts to the changes of the defect, since the device 1 move in a longitudinal direction at the same time as the cross-sectional size of the second element 6 is reduced. Therefore, the rupture is not increased, which may be the case if instead the longitudinal section has a round cross-sectional shape, since a rupture normally has a more elongated non-round shape.

By providing the device 1 with this second element 6, the device 1 or at least the second element 6, comprising braided material, can be made more flexible and thereby the device 1 will be less prone to increase the rupture size of an opening, when used to seal the opening. Furthermore, the device 1 can fill the gap of an opening completely to eliminate residual shunts or channels. Such residual shunts or channels may occur, when the opening is a rupture. Moreover, there is less radial strength in the second element 6 than in the first element 2, since the second element 6 is made of braided material. Another contributing reason for less radial strength in the second element 6 could be that the diameter of the thread or wire 9 can be made smaller than the diameter of the first element 2. Yet another contributing factor to the lower radial strength in the second element 6 could be due to the use of a different material, i.e. the material used for the first element 2 may be stiffer than a material used for the second element 6. Furthermore, by providing the device 1 with this second element 6, part of the device 1, i.e. the second element 6, can be made to have lower expansive force than the first element 2.

In some examples, the device 1 is also equipped with an adapter 14 for connecting the device 1 to a wire for delivery and/or retrieval. The first element 2 of the device can be formed substantially as a cone with the centre of the cone extending slightly inwardly towards the centre of the device. Thus, the adapter 14 is sunk in into the device 1 and will not impede blood flow in the vessel, where it is situated.

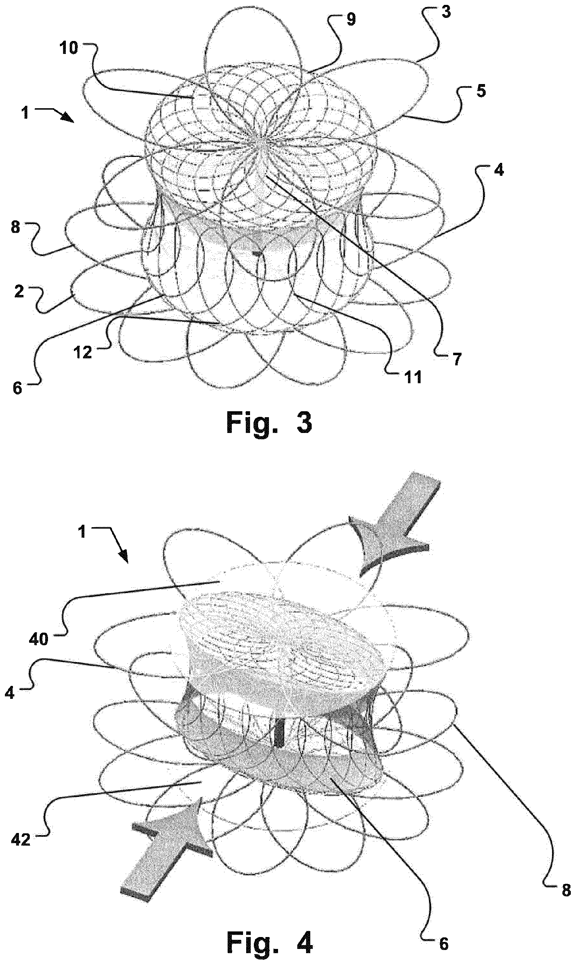

From Figure 3 it can be seen that the braided material is braided so that the at least one thread or wire 9 of the braided material has a certain first braid pitch in a first section 10, i.e. an end section, of the second element 6. From Figure 3, it can also be seen that in a second section 11, i.e. a middle section, of the second element 6, a certain second braid pitch, different from the first braid pitch, has been used. Thus, the second section 11, i.e. the middle section, of the second element 6 can be made more flexible than the first section of the second element 6, and thereby facilitating stretching of the second section 11. The second braid pitch can be larger or smaller than the first braid pitch. In order to make the second section 11 of the second element more flexible than the first section 10 of the second element, instead of using a different braid pitch, a lower number of threads or wires 9 can be used for the second section 11 of the second element than for the first section 10 of the second element.

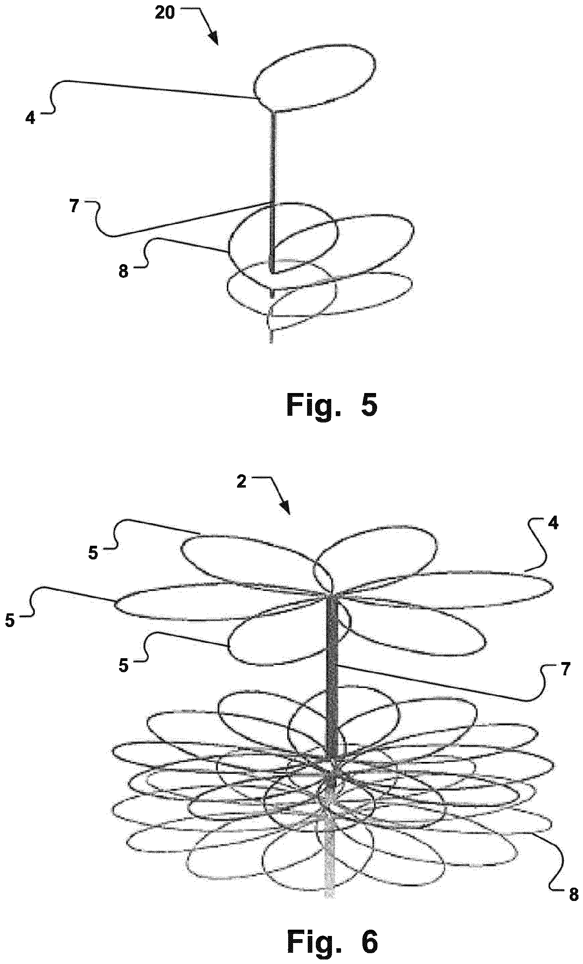

The first element 2 can further comprise a second section 7, such as a tubular section, which is connected to the first section 4. The second section 7 of the first element 2 is shown in Figure 6. The first section 4 has a larger diameter than a diameter of the second section 7.

The second element 6 does neither tear a body opening nor further tear an already torn body opening, since the second element 6 can be compressed in a different way than the first element 2 and since the second element 6, with braided material, can be compressed without deforming the first element 2.

By assembling a device from different, separately designed elements (first 2 and second 6), the strength of the disc-shaped section of the first element 2 does not affect the strength of the second element 6 and the second element 6 can stay oval with a very small retention force, which force can be supplied solely by the use of a material, which has shape memory properties.

Furthermore, by assembling a device from different elements (first 2 and second 6), an elastic design, which is suitable for both sistolic and diastolic thickness of the ventricular septum can be provided.

Optionally, the first element 2 may further comprise a third section 8, such as a non-braided disc-shaped section. The third section 8 of the first element is then connected to the second section 7 of the first element 2 and this third section 8 comprises at least one loop 5.

If the first element 2 of the device 1 comprises a third section 8, then both of the disc-shaped sections, i.e. the first section 2 and the third section 8, of the first element 2 can be larger, i.e. have a larger diameter and still be made up of less material, which is advantageous, since the material cost will be decreased and since healthy tissue will not be covered by the device 1 to the same extent as prior art devices.

Furthermore, in some examples, at least one loop 5 of the first section 4 is shaped as a petal and at least one loop 5 of the third section 8 is shaped as a petal. All of the loops 5 may be shaped as petals.

Moreover, in some examples at least one thread or wire 3 is used for the first, second and third sections 4, 7, 8 of the first element 2, so that the at least one thread or wire 3 runs through the first, second and third sections 4,7,8 of the first element 2.

This is illustrated in Figures 5 and 6. Figure 5 shows at the top, the first section 4 of the first element 2. The second section 7 and the third section 8 can also be seen from Figure 5. The first section 4 is connected to the second section 7 and the second section 7 is connected to the third section 8. In Figure 5, the first section 4 comprises only one loop or petal, whereas the third section comprises two overlapping loops or petals. However, any number of loops or petals can be used in the first and the third sections. In the example depicted in Figure 5, a single, sufficiently long thread or wire is used for all of the different sections 4, 7, 8, i.e. only one thread or wire is used to produce the part, comprising the first, second and third sections 4, 7, 8 of the first element 2.

From Figure 6, another example is shown, in which the first section 4 of the first element 2 can be seen at the top. The second section 7 and the third section 8 can also be seen in Figure 6. The first section 4 is connected to the second section 7 and the second section 7 is connected to the third section 8. In Figure 6, the first section 4 comprises a plurality of loops 5 or petals, whereas the third section 8 comprises another plurality of overlapping loops 5 or petals. Any number of loops 5 or petals can be used in the first and the third sections 4, 8 and the loops or petals may or may not be overlapping each other. In an example depicted in Figure 6, a single, sufficiently long thread or wire can be used for all of the different sections 4,7,8, i.e. only one thread or wire is needed to produce the first element 2, comprising the first, second and third sections 4,7,8. However, in another example, the first element 2 can also be assembled from different parts, such as the part 20 shown in Figure 5. As shown in Figure 5, the part 20 may comprising parts of the first, second and third sections 4, 7, 8 of the first element 2. In this example, the plurality of loops 5 are formed from a plurality of threads or wires, and the plurality of threads or wires are joined by a technique, such as welding, pinching the plurality of threads or wires together, clamping the plurality of threads or wires together, or hooking the plurality of threads or wires together. By using a plurality of threads or wires in order to manufacture different parts, such as the part 20 in Figure 5, and thereafter putting the different parts together, so that they will form a complete first element 2, manufacturing is simplified.

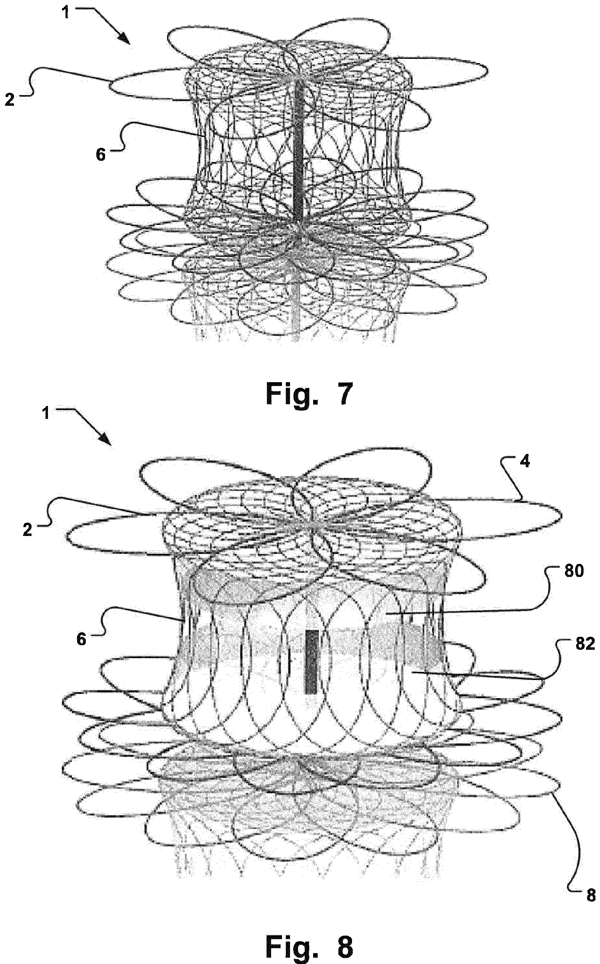

Figure 7 shows a device 1 for occluding an opening in a body. In this Figure, the first element 2 has been joined with the second element 6.

In one example, an end of the first element 2 is joined with a corresponding end of the second element 6 by a technique, such as welding, pinching the ends together, or hooking the ends together.

In another example, also another end of the first element 2 is joined with a corresponding end of the second element 6 by a technique, such as welding, pinching the ends together, clamping the ends together or hooking the ends together.

In some examples the first element 2 is joined with the second element 6 in an axial centre or a hub of the first and second elements 2, 6. By joining the first and second elements 2, 6 only in an axial centre or hub, the second element 6 can retain its flexibility.

In further examples, at least one element 2, 6 comprises a coating, such as accell™. The use of accell™ or other similar coatings enhances the adhesion and proliferation of fibroblasts and endothelial cells onto surfaces. It may also overcome certain materials cytotoxic issues. The use of accell™ or similar coatings may further provide faster endothelization.

In Figure 4, which is another view of the device 1 seen from the top at an angle, it can be seen what happens with the device 1, when radial pressure is applied from two directions, indicated with the arrows. When radial pressure is applied to the second element 6 of the device 1, the second element 6 will be deformed. Even if very little pressure is applied to the second element 6, it will be deformed, since the second element has been made very flexible, by the use of braided material, small diameter of the thread or wire, use of certain material or any combination of these methods. However, the first element 2 is not affected by the radial pressure applied, since the second element 6 is only attached to or joined with the first element 2 in an axial centre, at the ends of the second element 6 in this example.

From Figure 4, membranes 40,42 can be seen. In the example depicted in Figure 4, the membranes are located on the outside of the first and third sections 4, 8 of the first element 2, i.e. one membrane 40 at the top of the device 1 and one membrane at the bottom 42 of the device. In an alternative example, the membranes 40, 42 could instead be located on the other side of the first and third sections 4, 8 of the first element 2, i.e. one membrane wedged between the first section 4 of the first element 2 and the second element 6 and the other membrane 42 wedged between the third section 8 of the first element 2 and the second element 6. Alternatively, only one of the membranes 40,42 may be used. As another alternative, the membranes 40,42 may be attached to the inside of the first and third sections 4, 8 of the first element 2, i.e. a membrane 40 may be attached to the first section 4 from below in Figure 4 and a membrane 42 may be attached to the third section 8 from above in Figure 4.

Figure 8 shows another view of the device 1, in which membranes 80, 82 are located inside the second element 6. In this example one membrane 80 is located slightly closer to the first section 4 of the first element 2 than the other membrane 82. The membrane 80 can be located close to the end of the second element 6 that faces the first section 4 of the first element 2 or close to the middle of the second element 6. Likewise, the membrane 80 can be located close to the end of the second element 6 that faces the third section 8 of the first element 2 or close to the middle of the second element 6. The membrane 80 may also be located at a certain distance from the membrane 82. In some examples, only one of the membranes 80,82 may be used.

The membranes 40, 42, 80, 82 of the device 1 for occluding an opening in a body are preferably formed of a thin, flexible material, such as a fabric which may be folded taut without being damaged. Elastic polymeric materials such as nylon, polyester, polypropylene, polytetrafluoroethylene and expanded polytetrafluoroethylene, as well as natural fabrics such as silk or wool, should meet the requirements of the membrane. In one example which has been found to work well, the membranes 40, 42, 80, 82 are formed of a woven polyester. The membranes 40, 42, 80,82 can also be made, at least partly, by a biodegradable material. The membranes 40, 42, 80, 82 facilitate thrombosis, since they are made of a dense material. The membranes may also provide an improved endothelialization.

Referring again to Figure 3, the first section 4 of the first element 2 comprises a plurality of loops 5 in a first geometrical plane, which loops 5 are arranged in a regular pattern, with each loop 5 extending from an axial centre of the first geometrical plane, and each loop 5 being adjacent to another loop 5 on a first side in the first geometrical plane and also adjacent to another loop 5 on a second side in the first geometrical plane, so that the plurality of loops 5 together form the shape of a corolla. Further, from Figure 3 it can also be seen that in one example, the third section 8 of the first element 2 comprises a plurality of loops 5 in a second geometrical plane, which loops 5 are arranged in a regular pattern, with each loop 5 extending from an axial centre of the second geometrical plane, and each loop 5 being adjacent to another loop 5 on a first side in the second geometrical plane and also adjacent to another loop 5 on a second side in the second geometrical plane, so that the plurality of loops 5 together form the shape of a corolla. By forming the first and third sections 4, 8 of the first element this way, a symmetrical design of the first section 4 as well as the third section 8 is achieved. In one example, the first geometrical plane is parallel with the second geometrical plane.

The plurality of loops 5 of the first section 4 of the first element 2 are, in one example, overlapping in the first geometrical plane. In another example, the plurality of loops 5 of the third section 8 of the first element 2 are overlapping in the second geometrical plane. Alternatively, the loops 5 in both the first and the second geometrical planes are overlapping.

In some examples, the plurality of loops 5 of the first section 4 are fewer than the plurality of loops 5 of the third section 8.

In some examples, the diameter of the second element 6 is smaller than the diameter of the first section 4 of the first element 2. This is advantageous, since the first section 4, when used, is preferably on the side of a septum or body wall.

In some examples, the diameter of the second element 6 is smaller than the diameter of the third section 8 of the first element 2. This is advantageous, since the third section 8, when used, is preferably on the side of a septum or body wall.

Alternatively, both the diameter of the first section 4 and the diameter of the third section 8 are larger than the diameter of the second element 6.

In one example, the diameter of the first section 4 of the first element 2 is larger than a diameter of the third section 8 of the first element 2. This may be advantageous in some situations, when a rupture needs to be sealed at a target site, where the space on one side of the rupture is more limited than on the other side of the rupture.

In some examples, the plurality of loops 5 are formed from one single thread or wire 3, Alternatively, the plurality of loops 5 are formed from a plurality of threads or wires 3, whereafter the plurality of threads or wires 3 are joined by a technique, such as welding. Other techniques for joining the threads or wires 3, such as pinching the plurality of threads or wires together, clamping the plurality of threads or wires together, or hooking the plurality of threads or wires together, could be used. By first forming the loops 5 from a plurality of threads or wires 3 and then joining the plurality of threads or wires 3, the manufacturing is simplified.

One example of this disclosure is a method of manufacturing a device 1 for occluding an opening in a body. This method comprises forming a first section 4 of a first element 2, or part thereof by forming at least one loop 5 from a single thread or wire 3 in a first geometrical plane. The method further comprises forming a second section 7 of a first element 2, or part thereof by extending the single thread or wire 3 perpendicularly from the first geometrical plane to a second geometrical plane, the second geometrical plane preferably being parallel to the first geometrical plane. Another step of the method is to form a third section 8 of the first element 2, or part thereof by forming at least one loop 5 from the single thread or wire 3 in the second geometrical plane. An option of the method is to join different parts 20 by a technique, such as welding, pinching the plurality of threads or wires 3 together, clamping the plurality of threads or wires 3 together, or hooking the plurality of threads or wires 3 together, each part 20 comprising parts of the first, second and third sections 4, 7, 8 of the first element 2, if only parts of the first, second and third sections 4, 7, 8 were formed in the previous steps. In the method, forming of a first section 10 of a second element 6 by braiding at least one thread or wire 9 with a first braid pitch may also be performed. Also forming of a second section 11 of a second element 6 by braiding at least one thread or wire 9 with a second braid pitch, which braid pitch is smaller than the first braid pitch is performed. A third section 12 of a second element 6 is also formed by braiding at least one thread or wire 9 with the first braid pitch. The first and second elements 2, 6 are thereafter joined by a technique, such as welding, pinching the plurality of threads or wires together, clamping the plurality of threads or wires together, or hooking the plurality of threads or wires together.

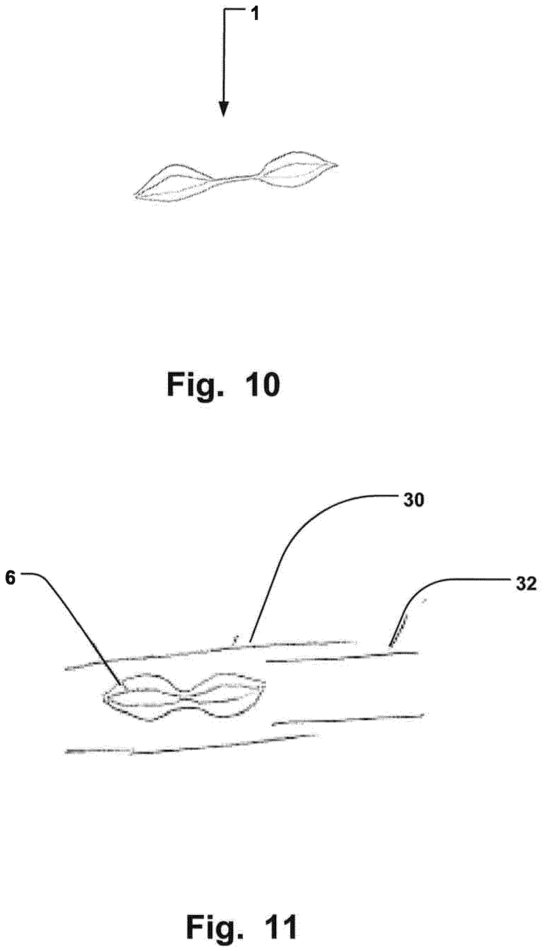

Another example of this disclosure is a medical procedure for occluding an opening in a body. The catheters 30, 32 used in the medical procedure and the device 1 are shown in Figure 11. The medical procedure comprises positioning of a device 1 inside a restraining catheter 30. The medical procedure also comprises positioning of a pushing catheter 32 inside the restraining catheter 30 adjacent to the device 1, further away from a target site than the device 1. Thereafter the restraining catheter 30, the pushing catheter 32 and the device 1 are inserted into the body. A distal end of the restraining catheter 30 is positioned at the target site and a device 1 is positioned inside the body opening. Thereafter the device 1 is pushed through the restraining catheter 30 with the pushing catheter 32 until the device 1 has been released, so that a first section 4 of a first element 2 of the device 1 is positioned on an inside of a rupture to be sealed. Another step of the medical procedure involves removing of the pushing catheter 32. Thereafter, the restraining catheter 30 is removed, so that the first section 4 of the first element 2 of the device 1 is positioned on the inside of the rupture to be sealed with the device 1 and a third section 8 of the first element 2 is positioned on an outside of the rupture, whereby a second element 6 of the device 1 is returned to its preset shape and the device 1 thereby is radially contracted so as to close the rupture. The restraining catheter 30 can thereafter be removed from the body.

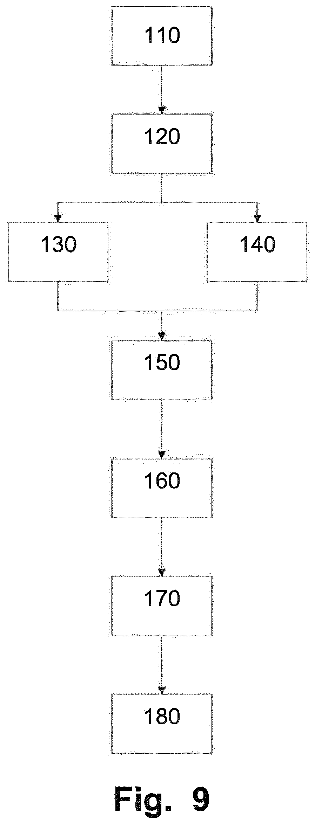

A further example of the disclosure is illustrated in Figure 9. Figure 9 shows the steps of a method of manufacturing a device 1 for occluding an opening in a body. In step 110, a first section 4 of a first element 2, or part thereof is formed by forming at least one loop 5 from a single thread or wire 3 in a first geometrical plane. In step 120 a second section 7 of a first element 2, or part thereof is formed by extending the single thread or wire 3 perpendicularly from the first geometrical plane to a second geometrical plane. The second geometrical plane is preferably parallel to the first geometrical plane. In step 130 a third section 8 of the first element 2, or part thereof is formed by forming at least one loop 5 from the single thread or wire 3 in the second geometrical plane. If only parts of the first and third sections 4, 8 were formed in steps 110 and 130, then the different parts 20 are joined by a technique, such as welding, pinching the plurality of threads or wires together, clamping the plurality of threads or wires together, or hooking the plurality of threads or wires together. This is shown in Figure 9 with the step 140. In step 150, a first section 10 of a second element 6 is formed by braiding at least one thread or wire 9 with a first braid pitch. A second section 11 of a second element 6 is formed in step 160 by braiding at least one thread or wire 9 with a second braid pitch. The second braid pitch can be smaller than the first braid pitch. The second braid pitch could also be larger than the first braid pitch. In step 170 a third section 12 of a second element 6 is formed by braiding at least one thread or wire 9 with the first braid pitch. Thereafter, in step 180, the first and second elements 2, 6 are joined by a technique, such as welding, pinching the plurality of threads or wires together, clamping the plurality of threads or wires together, or hooking the plurality of threads or wires together. Instead or in addition to having different braid pitch for the different sections of the second element 6, the second element may have fewer threads, wires for the longitudinal section than for the end sections, i.e. the second section 11 of the second element 6 may comprise fewer threads or wires 9 than the first and the third sections 10, 12 of the second element 6.

Figure 10 shows the device 1 for occluding a body opening in its collapsed state. The device 1 is, preferably made of a shape memory material, such as nitinol. This is advantageous, since the device can then take on either a preset expanded configuration or a collapsed configuration. The device 1 can then be configured to be constrained to respective collapsed configuration for delivery to the target site and to at least partially return to the respective preset, expanded configuration at the target site when unconstrained.

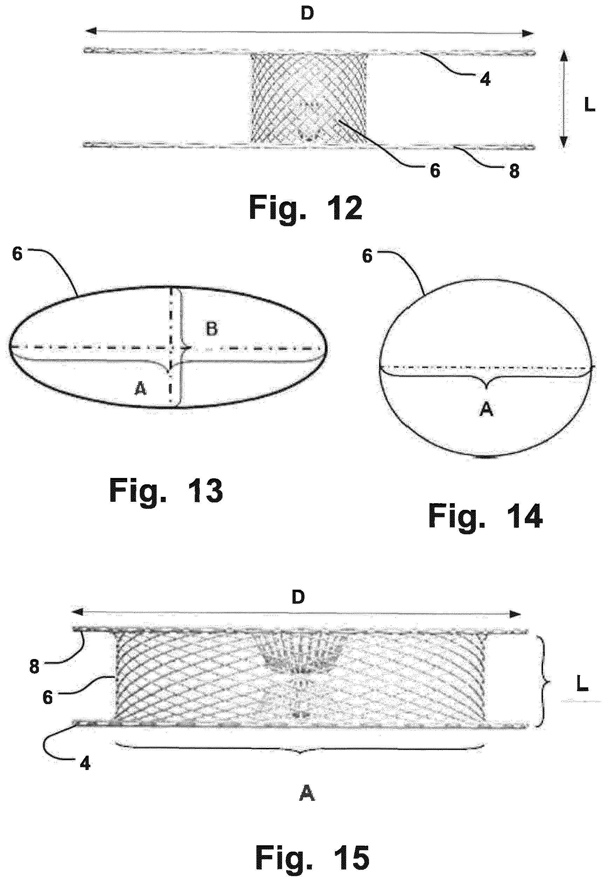

In some examples, the occluder is used for a postinfarction ventricular septal defect. This example is depicted in Figs. 12 and 13. As can be seen from Fig. 13, the second element 6 has an oval-like shape or is shaped as an oval. In Fig. 13, the shorter diameter is denoted B, whereas the longer diameter is denoted A. As can be seen from Fig. 12, the distance between the first section 4 and the third section 8 of the first element 2, when the device is in its preset expanded configuration, is denoted L and the diameter of the first section 4 or the third section 8 of the first element 2 is denoted D. The ratio of diameter A to diameter B is preferably in the range of 1.8-5.15, whereas the ratio of the diameter D to the distance L is in the range of 1.4-5.6 and preferably in the range of 3.2-5.6. Furthermore, the ratio of D to A is preferably in the range of 1.3-2.2 and the ratio of D to B in the range of 2.3-6.3. An occluder having the above given ratios is particularly well suited for being used for a postinfarction ventricular septal defect. In this example, the distance between the first section 4 and the third section 8 of the first element 2 is preferably of a fixed size, but can be stretched according to the septum thickness.

In some examples, the occluder is used for a muscular ventricular septal defect. As can be seen from Fig. 14, the second element 6 has a circle-like shape or is shaped as a circle. In Figs. 14 and 15, the diameter of the second element 6 is denoted A, whereas the diameter of the first section 4 or the third section 8 of the first element 2 is denoted D. The distance between the first section 4 and the third section 8 of the first element 2, when the device is in its preset expanded configuration, is denoted L. The ratio of A to D is preferably in the range of 0.14-2 whereas the ratio of the distance L to A preferably is in the range of 0.35-1.75. Furthermore, the ratio of D to L is in the range of 1.4-5.6 and preferably in the range of 1.4-4. An occluder having the above given ratios is particularly well suited for being used for a muscular ventricular septal defect. For the Muscular VSD, the distance between the first section 4 and the third section 8 of the first element 2 is preferably of a fixed size, but can be stretched. This distance can also be shorter, and may then eliminate residual shunts or channels.

The present disclosure has been described above with reference to specific examples. However, other examples than the above described are equally possible within the scope of the disclosure. Different method steps than those described above, may be provided within the scope of the disclosure. The different features and steps of the disclosure may be combined in other combinations than those described. The scope of the disclosure is only limited by the appended patent claims. More generally, those skilled in the art will readily appreciate that all parameters, dimensions, materials, and configurations described herein are meant to be exemplary-insofar they are not required by the independent claims -- and that the actual parameters, dimensions, materials, and/or configurations will depend upon the specific application or applications for which the teachings of the present disclosure is/are used.

## Claims

1. A device (1) for occluding an opening in the heart, comprising:
a first element (2), comprising at least one thread or wire (3), and
a second element (6), which is joined with the first element (2) and further comprising at least one longitudinal section, said second element (6) comprising a braided material, said braided material comprising at least one thread or wire (9), and wherein a diameter of said second element (6) is of substantially the same size or smaller than said opening for all of the at least one longitudinal sections, when said device (1) is in a preset, expanded configuration, wherein
the first element (2) has a first section (4) and a third section (8) with a distance denoted L between the first section (4) and the third section (8) of the first element (2), when the device is in its preset expanded configuration, and with a diameter denoted D of the first section (4) or the third section (8) of the first element (2),
said second element (6) has an oval-like shape in its cross-section with a shorter diameter denoted B, and a longer diameter denoted A, **characterized in that**
a ratio of said longer diameter denoted A to said shorter diameter denoted B is in the range of 1.8-5.15,
a ratio of said diameter denoted D to said distance denoted L is in the range of 1.4-5.6, preferably in the range of 3.2-5.6;
a ratio of said diameter denoted D to said longer diameter denoted A is in the range of 1.3-2.2; and
a ratio of said diameter denoted D to said shorter diameter denoted B is in the range of 2.3-6.3.

2. The device of any of claims 1, wherein said braided material is braided so that said at least one thread or wire (9) of said braided material has a certain first braid pitch in a first section (10) of said second element (6) and a certain second braid pitch, different from said first braid pitch, in a second section (11) of said second element (6).

3. The device of claim 2, wherein said first braid pitch is larger than said second braid pitch.

4. The device of any of claims 1-3, wherein at least one element (2, 6) comprises a coating for enhancing the adhesion and proliferation of fibroblasts and endothelial cells onto surfaces.

5. The device of any of claims 1-4, wherein said thread or wire (3, 9) of said first element (2) and/or second element (6) has a diameter that varies along its length.

6. The device of any of claims 1-5, wherein said thread or wire (3, 9) of said first element (2) and second element (6) each comprises a shape memory material having an initial, expanded configuration corresponding to a preset, expanded configuration, and wherein said thread or wire (3, 9) of said first element (2) and second element (6) are configured to be constrained from their preset, expanded configurations to their reduced configurations for delivery to the target site and to self-expand and at least partially return to their respective preset, expanded configurations at a target site when unconstrained.

7. The device of any of claims 1-6, wherein said first element (2) of said device is formed substantially as a cone with the centre of the cone extending slightly inwardly towards the centre of the device.

8. A method of manufacturing a device (1) for occluding an opening in the heart, comprising:
forming first element (2), or part thereof, of at least one thread or wire, with a first section (4) and a third section (8) with a distance denoted L between the first section (4) and the third section (8) of the first element (2), when the device is in its preset expanded configuration, and with a diameter denoted D of the first section (4) or the third section (8) of the first element (2);
forming at least one longitudinal section of a second element (6), which is joined with the first element (2), by braiding of at least one thread or wire so that a diameter of said second element (6) is of substantially the same size or smaller than said opening for all of the at least one longitudinal sections, when said device (1) is in a preset, expanded configuration; wherein
forming said second element to have an oval-like shape in its cross-section with a shorter diameter denoted B, and a longer diameter denoted A, **characterized by** forming said first element (2) and said second element (6) such that
a ratio of said longer diameter denoted A to said shorter diameter denoted B is in the range of 1.8-5.15,
a ratio of said diameter denoted D to said distance denoted L is in the range of 1.4-5.6, preferably in the range of 3.2-5.6;
a ratio of said diameter denoted D to said longer diameter denoted A is in the range of 1.3-2.2; and
a ratio of said diameter denoted D to said shorter diameter denoted B is in the range of 2.3-6.3.

9. The method of manufacturing a device (1) according to claim 8 further comprising, joining said first and second elements (2, 6).

10. The method of manufacturing a device (1) according to claims 8-9,
comprising forming a first section (10) of said second element (6) is performed by braiding at least one thread or wire (9) with a first braid pitch, and
forming a second section (11) of said second element (6) is performed by braiding at least one thread or wire (9) with a second braid pitch, said second braid pitch being smaller than said first braid pitch.

11. The method of manufacturing a device (1) according to claims 8-10, comprising forming a third section (12) of said second element (6) is performed by braiding at least one thread or wire (9) with said first braid pitch.

12. The method of manufacturing a device (1) according to claims 9, wherein said joining said first and second elements (2, 6) is performed by a technique, such as welding, pinching said plurality of threads or wires together, clamping said plurality of threads or wires together, or hooking said plurality of threads or wires together.

13. The method of manufacturing a device (1) according to claims 8-12, wherein at least one element (2, 6) comprises a coating for enhancing the adhesion and proliferation of fibroblasts and endothelial cells onto surfaces.

14. The method of manufacturing a device (1) according to claims 8-13, wherein said thread or wire (3, 9) of said first element (2) and second element (6) each comprises a shape memory material having an initial, expanded configuration corresponding to a preset, expanded configuration, and wherein said thread or wire (3, 9) of said first element (2) and second element (6) are configured to be constrained from their preset, expanded configurations to their reduced configurations for delivery to the target site and to self-expand and at least partially return to their respective preset, expanded configurations at a target site when unconstrained.

15. The method of manufacturing a device (1) according to claims 8-14, wherein said first and/ or second element is manufactured of one thread.

## Patentansprüche

1. Vorrichtung (1) zur Okklusion einer Öffnung im Herzen, umfassend:
erstes Element (2), das mindestens eine Schnur oder einen Draht (3) umfasst, und
zweites Element (6), das mit dem ersten Element (2) zusammengefügt ist und ferner mindestens einen längsverlaufenden Abschnitt umfasst, wobei das zweite Element (6) ein geflochtenes Material umfasst, wobei das geflochtene Material mindestens eine Schnur oder einen Draht (9) umfasst, und wobei ein Durchmesser des zweiten Elements (6) für alle der mindestens einen längsverlaufenden Abschnitte im Wesentlichen dieselbe Größe oder eine geringere Größe aufweist als die Öffnung, wenn sich die Vorrichtung (1) in einer voreingestellten expandierten Konfiguration befindet, wobei
das erste Element (2) einen ersten Abschnitt (4) und einen dritten Abschnitt (8) aufweist, wobei ein Abstand zwischen dem ersten Abschnitt (4) und dem dritten Abschnitt (8) des ersten Elements (2), wenn sich die Vorrichtung in ihrer voreingestellten expandierten Konfiguration befindet, mit L bezeichnet ist, und wobei ein Durchmesser des ersten Abschnitts (4) oder des dritten Abschnitts (8) des ersten Elements (2) mit D bezeichnet ist,
wobei das zweite Element (6) in seinem Querschnitt eine ovalartige Form aufweist, wobei ein kürzerer Durchmesser mit B bezeichnet ist, und ein längerer Durchmesser mit A bezeichnet ist, **dadurch gekennzeichnet, dass**
ein Verhältnis des längeren Durchmessers, der mit A bezeichnet ist, zu dem kürzeren Durchmesser, der mit B bezeichnet ist, im Bereich von 1,8 bis 5,15 liegt,
ein Verhältnis des Durchmessers, der mit D bezeichnet ist, zu dem Abstand, der mit L bezeichnet ist, im Bereich von 1,4 bis 5,6, vorzugsweise im Bereich von 3,2 bis 5,6, liegt;
ein Verhältnis des Durchmessers, der mit D bezeichnet ist, zu dem längeren Durchmesser, der mit A bezeichnet ist, im Bereich von 1,3 bis 2,2 liegt; und
ein Verhältnis des Durchmessers, der mit D bezeichnet ist, zu dem kürzeren Durchmesser, der mit B bezeichnet ist, im Bereich von 2,3 bis 6,3 liegt.

2. Vorrichtung gemäß einem beliebigen der Ansprüche 1, wobei das geflochtene Material so geflochten ist, dass die mindestens eine Schnur oder der mindestens eine Draht (9) des geflochtenen Materials in einem ersten Abschnitt (10) des zweiten Elements (6) eine bestimmte erste Flechtsteigung aufweist, und in einem zweiten Abschnitt (11) des zweiten Elements (6) eine bestimmte zweite Flechtsteigung, welche sich von der ersten Flechtsteigung unterscheidet, aufweist.

3. Vorrichtung gemäß Anspruch 2, wobei die erste Flechtsteigung größer als die zweite Flechtsteigung ist.

4. Vorrichtung gemäß einem beliebigen der Ansprüche 1 bis 3, wobei mindestens ein Element (2, 6) eine Beschichtung zur Verbesserung der Haftung und der Proliferation von Fibroblasten und Endothelzellen auf Oberflächen umfasst.

5. Vorrichtung gemäß einem beliebigen der Ansprüche 1 bis 4, wobei die Schnur oder der Draht (3, 9) des ersten Elements (2) und/oder des zweiten Elements (6) einen Durchmesser aufweist, der sich entlang seiner Länge verändert.

6. Vorrichtung gemäß einem beliebigen der Ansprüche 1 bis 5, wobei jede Schnur oder jeder Draht (3, 9) des ersten Elements (2) und/oder des zweiten Elements (6) ein Formgedächtnismaterial umfasst, das eine anfängliche expandierte Konfiguration aufweist, die einer voreingestellten expandierten Konfiguration entspricht, und wobei die Schnur oder der Draht (3, 9) des ersten Elements (2) und des zweiten Elements (6) so eingerichtet sind, dass sie von ihren voreingestellten expandierten Konfigurationen in ihre reduzierten Konfigurationen zusammengepresst werden, um an die Zielstelle zugeführt zu werden und um an der Zielstelle selbst zu expandieren und zumindest teilweise in ihre jeweiligen voreingestellten expandierten Konfigurationen zurückzukehren, wenn sie nicht zusammengepresst werden.

7. Vorrichtung gemäß einem beliebigen der Ansprüche 1 bis 6, wobei das erste Element (2) der Vorrichtung im Wesentlichen als Kegel geformt ist, wobei der Mittelpunkt des Kegels sich leicht nach innen zu dem Mittelpunkt der Vorrichtung hin erstreckt.

8. Verfahren zur Herstellung einer Vorrichtung (1) zur Okklusion einer Öffnung im Herzen, umfassend:
Formung des erstes Elements (2), oder einem Teil von diesem, von mindestens einer Schnur oder einem Draht, mit einem ersten Abschnitt (4) und einem dritten Abschnitt (8), wobei ein Abstand zwischen dem ersten Abschnitt (4) und dem dritten Abschnitt (8) des ersten Elements (2) mit L bezeichnet ist, wenn sich die Vorrichtung in ihrer voreingestellten expandierten Konfiguration befindet, und wobei ein Durchmesser des ersten Abschnitts (4) oder des dritten Abschnitts (8) des ersten Elements (2) mit D bezeichnet ist;
Formung des mindestens einen längsverlaufenden Abschnitts des zweiten Elements (6), das mit dem ersten Element (2) durch Flechten der mindestens einen Schnur oder des mindestens einen Drahtes (9) derart zusammengefügt ist, dass ein Durchmesser des zweiten Elements (6) für alle der mindestens einen längsverlaufenden Abschnitte im Wesentlichen dieselbe Größe oder eine geringere Größe aufweist als die Öffnung, wenn sich die Vorrichtung (1) in einer voreingestellten expandierten Konfiguration befindet, wobei
das zweite Element so geformt ist, dass es in seinem Querschnitt eine ovalartige Form aufweist, wobei ein kürzerer Durchmesser mit B bezeichnet ist, und ein längerer Durchmesser mit A bezeichnet ist, **dadurch gekennzeichnet, dass** das erste Element (2) und das zweite Element (6) so geformt sind, dass
ein Verhältnis des längeren Durchmessers, der mit A bezeichnet ist, zu dem kürzeren Durchmesser, der mit B bezeichnet ist, im Bereich von 1,8 bis 5,15 liegt,
ein Verhältnis des Durchmessers, der mit D bezeichnet ist, zu dem Abstand, der mit L bezeichnet ist, im Bereich von 1,4 bis 5,6, vorzugsweise im Bereich von 3,2 bis 5,6, liegt;
ein Verhältnis des Durchmessers, der mit D bezeichnet ist, zu dem längeren Durchmesser, der mit A bezeichnet ist, im Bereich von 1,3 bis 2,2 liegt; und
ein Verhältnis des Durchmessers, der mit D bezeichnet ist, zu dem kürzeren Durchmesser, der mit B bezeichnet ist, im Bereich von 2,3 bis 6,3 liegt.

9. Verfahren zur Herstellung einer Vorrichtung (1) gemäß Anspruch 8, ferner das Zusammenfügen des ersten und des zweiten Elements (2, 6) umfassend.

10. Verfahren zur Herstellung einer Vorrichtung (1) gemäß den Ansprüchen 8 bis 9, die Formung eines ersten Abschnitts (10) des zweiten Elements (6) umfassend, wobei diese mittels des Flechtens mindestens einer Schnur oder mindestens eines Drahtes (9) mit einer ersten Flechtsteigung durchgeführt wird, und
Formung eines zweiten Abschnitts (11) des zweiten Elements (6) umfassend, wobei diese mittels des Flechtens mindestens einer Schnur oder mindestens eines Drahtes (9) mit einer zweiten Flechtsteigung durchgeführt wird, wobei die zweite Flechtsteigung geringer als die erste Flechtsteigung ist.

11. Verfahren zur Herstellung einer Vorrichtung (1) gemäß den Ansprüchen 8 bis 10, die Formung eines dritten Abschnitts (12) des zweiten Elements (6) umfassend, wobei diese mittels des Flechtens mindestens einer Schnur oder eines Drahtes (9) mit der ersten Flechtsteigung durchgeführt wird.

12. Verfahren zur Herstellung einer Vorrichtung (1) gemäß den Ansprüchen 9, wobei das Zusammenfügen des ersten und zweiten Elements (2,6) mittels einer Technik, wie beispielsweise durch Verschweißen, Verklemmen der Vielzahl von Schnüren oder Drähten (9), Zusammenklammern der Vielzahl von Schnüren oder Drähten, oder Zusammenhaken der Vielzahl von Schnüren oder Drähten durchgeführt wird.

13. Verfahren zur Herstellung einer Vorrichtung (1) gemäß den Ansprüchen 8 bis 12, wobei mindestens ein Element (2,6) eine Beschichtung zur Verbesserung der Haftung und der Proliferation von Fibroblasten und Endothelzellen auf Oberflächen umfasst.

14. Verfahren zur Herstellung einer Vorrichtung (1) gemäß den Ansprüchen 8 bis 13, wobei jede Schnur oder jeder Draht (3, 9) des ersten Elements (2) und des zweiten Elements (6) ein Formgedächtnismaterial umfasst, das eine anfängliche expandierte Konfiguration aufweist, die einer voreingestellten expandierten Konfiguration entspricht, und wobei die Schnur oder der Draht (3, 9) des ersten Elements (2) und des zweiten Elements (6) so eingerichtet sind, dass sie aus ihren voreingestellten expandierten Konfigurationen in ihre reduzierten Konfigurationen zusammengepresst werden, um an die Zielstelle zugeführt zu werden und um an der Zielstelle selbst zu expandieren und zumindest teilweise in ihre jeweiligen voreingestellten expandierten Konfiguration zurückzukehren, wenn sie nicht zusammengepresst werden.

15. Verfahren zur Herstellung einer Vorrichtung (1) gemäß den Ansprüchen 8 bis 14, wobei das erste und/oder zweite Element aus einer einzigen Schnur hergestellt ist.

## Revendications

1. Dispositif (1) d'occlusion d'une ouverture dans le coeur, comprenant :
un premier élément (2), comprenant au moins un fil ou fil métallique (3), et
un deuxième élément (6), lequel est relié au premier élément (2) et comprenant en outre au moins une section longitudinale, ledit deuxième élément (6) comprenant un matériau tressé, ledit matériau tressé comprenant au moins un fil ou fil métallique (9), et où un diamètre dudit deuxième élément (6) est quasiment de la même taille ou est plus petit que ladite ouverture sur toutes les au moins une sections longitudinales, lorsque ledit dispositif (1) se trouve dans une configuration déployée prédéfinie, où
le premier élément (2) présente une première section (4) et une troisième section (8), une distance notée L séparant la première section (4) et la troisième section (8) du premier élément (2), lorsque le dispositif se trouve dans sa configuration déployée prédéfinie, et la première section (4) ou la troisième section (8) du premier élément (2) présentant un diamètre noté D,
ledit deuxième élément (6) présente une forme ovale dans sa section transversale ayant un diamètre plus court noté B, et un diamètre plus long noté A, **caractérisé en ce que**
un rapport dudit diamètre plus long noté A sur ledit diamètre plus court noté B est compris dans la plage allant de 1,8 à 5,15,
un rapport dudit diamètre noté D sur ladite distance notée L est compris dans la plage allant de 1,4 à 5,6, de préférence dans la plage allant de 3,2 à 5,6 ;
un rapport dudit diamètre noté D sur ledit diamètre plus long noté A est compris dans la plage allant de 1,3 à 2,2 ; et
un rapport dudit diamètre noté D sur ledit diamètre plus court noté B est compris dans la plage allant de 2,3 à 6,3.

2. Dispositif selon la revendication 1, dans lequel ledit matériau tressé est tressé de sorte que ledit au moins un fil ou fil métallique (9) constitué dudit matériau tressé présente un certain premier pas d'enroulement dans une première section (10) dudit deuxième élément (6) et un certain deuxième pas d'enroulement, différent dudit premier pas d'enroulement, dans une deuxième section (11) dudit deuxième élément (6).

3. Dispositif selon la revendication 2, dans lequel ledit premier pas d'enroulement est plus important que ledit deuxième pas d'enroulement.

4. Dispositif selon l'une quelconque des revendications 1 à 3, dans lequel au moins un élément (2, 6) comprend un revêtement destiné à améliorer l'adhérence et la prolifération de fibroblastes et de cellules endothéliales sur des surfaces.

5. Dispositif selon l'une quelconque des revendications 1 à 4, dans lequel ledit fil ou fil métallique (3, 9) dudit premier élément (2) et/ou dudit deuxième élément (6) présente un diamètre qui varie sur sa longueur.

6. Dispositif selon l'une quelconque des revendications 1 à 5, dans lequel lesdits fils ou fils métalliques (3, 9) dudit premier élément (2) et dudit deuxième élément (6) comprennent chacun un matériau à mémoire de forme ayant une configuration déployée initiale correspondant à une configuration déployée prédéfinie, et où lesdits fils ou fils métalliques (3, 9) dudit premier élément (2) et dudit deuxième élément (6) sont configurés pour être comprimés de sorte à passer de leurs configurations déployées prédéfinies à leurs configurations rétractées en vue d'une pose sur le site cible et pour se déployer d'eux-mêmes et retourner au moins partiellement à leurs configurations déployées prédéfinies respectives au niveau d'un site cible lorsqu'ils ne sont plus comprimés.

7. Dispositif selon l'une quelconque des revendications 1 à 6, dans lequel ledit premier élément (2) dudit dispositif est façonné globalement sous le forme d'un cône, le centre du cône s'étendant légèrement vers l'intérieur en direction du centre du dispositif.

8. Procédé de fabrication d'un dispositif (1) d'occlusion d'une ouverture dans le coeur, comprenant les étapes consistant à :
former un premier élément (2), ou une partie de celui-ci, avec au moins un fil ou fil métallique, ayant une première section (4) et une troisième section (8), une distance notée L séparant la première section (4) et la troisième section (8) du premier élément (2), lorsque le dispositif se trouve dans sa configuration déployée prédéfinie, et la première section (4) ou la troisième section (8) du premier élément (2) présentant un diamètre noté D ;
former au moins une section longitudinale d'un deuxième élément (6), lequel est relié au premier élément (2) en enroulant au moins un fil ou fil métallique de sorte qu'un diamètre dudit deuxième élément (6) est quasiment de la même taille ou est plus petit que ladite ouverture pour toutes les au moins une sections longitudinales, lorsque ledit dispositif (1) se trouve dans une configuration déployée prédéfinie ; où
former ledit deuxième élément de sorte qu'il présente une forme ovale dans sa section transversale avec un diamètre plus court noté B, et un diamètre plus long noté A, **caractérisé par** le fait de former ledit premier élément (2) et ledit deuxième élément (6) de telle sorte que
un rapport dudit diamètre plus long noté A sur ledit diamètre plus court noté B est compris dans la plage allant de 1,8 à 5,15,
un rapport dudit diamètre noté D sur ladite distance notée L est compris dans la plage allant de 1,4 à 5,6, de préférence dans la plage allant de 3,2 à 5,6 ;
un rapport dudit diamètre noté D sur ledit diamètre plus long noté A est compris dans la plage allant de 1,3 à 2,2 ; et
un rapport dudit diamètre noté D sur ledit diamètre plus court noté B est compris dans la plage allant de 2,3 à 6,3.

9. Procédé de fabrication d'un dispositif (1) selon la revendication 8 comprenant en outre le fait de relier lesdits premier et deuxième éléments (2, 6).

10. Procédé de fabrication d'un dispositif (1) selon les revendications 8 et 9, comprenant le fait de former une première section (10) dudit deuxième élément (6) en enroulant au moins un fil ou fil métallique (9) selon un premier pas d'enroulement, et
le fait de former une deuxième section (11) dudit deuxième élément (6) en enroulant au moins un fil ou fil métallique (9) selon un deuxième pas d'enroulement, ledit deuxième pas d'enroulement étant plus petit que ledit premier pas d'enroulement.

11. Procédé de fabrication d'un dispositif (1) selon les revendications 8 à 10, comprenant le fait de former une troisième section (12) dudit deuxième élément (6) en enroulant au moins un fil ou fil métallique (9) selon ledit premier pas d'enroulement

12. Procédé de fabrication d'un dispositif (1) selon la revendication 9, dans lequel ladite étape consistant à relier lesdits premier et deuxième éléments (2, 6) est réalisée au moyen d'une technique, telle qu'un soudage, le fait de pincer ensemble ladite pluralité de fils ou fils métalliques, le fait d'attacher ensemble ladite pluralité de fils ou fils métalliques, ou le fait de crocheter ensemble ladite pluralité de fils ou fils métalliques.

13. Procédé de fabrication d'un dispositif (1) selon les revendications 8 à 12, dans lequel au moins un élément (2, 6) comprend un revêtement destiné à améliorer l'adhérence et la prolifération de fibroblastes et de cellules endothéliales sur des surfaces.

14. Procédé de fabrication d'un dispositif (1) selon les revendications 8 à 13, dans lequel lesdits fils ou fils métalliques (3, 9) dudit premier élément (2) et dudit deuxième élément (6) comprennent chacun un matériau à mémoire de forme ayant une configuration déployée initiale correspondant à une configuration déployée prédéfinie, et où lesdits fils ou fils métalliques (3, 9) dudit premier élément (2) et dudit deuxième élément (6) sont configurés pour être comprimés de sorte à passer de leurs configurations déployées prédéfinies à leurs configurations rétractées en vue d'une pose sur le site cible et pour se déployer d'eux-mêmes et retourner au moins partiellement à leurs configurations déployées prédéfinies respectives au niveau d'un site cible lorsqu'ils ne sont plus comprimés.

15. Procédé de fabrication d'un dispositif (1) selon les revendications 8 à 14, dans lequel ledit premier élément et/ou ledit deuxième élément sont fabriqués à partir d'un seul fil.
